# FASCICULE DE BREVET EUROPEEN

(11) **EP 2 086 479 B1**
(45) Date de publication et mention de la délivrance du brevet: **03.10.2012**
(21) Numéro de dépôt: 07866443.0
(22) Date de dépôt: 26.10.2007
(51) Int. Cl.: A61F 9/007

(54) **ENSEMBLE D'INTUBATION MONOCANALICULONASAL ET/OU MONOCANALICULAIRE, NOTAMMENT POUR L'IMPERFORATION LACRYMONASALE**
MONOCANALICULONASALE UND/ODER MONOCANALICULARE INTUBATIONSANORDNUNG FÜR NASOLAKRIMALE IMPERFORATION
MONOCANALICULONASAL AND/OR MONOCANALICULAR INTUBATION ASSEMBLY FOR NASOLACRIMAL IMPERFORATION

(30) Priorité: 08.11.2006 FR 0609736
(43) Date de publication de la demande: 12.08.2009
(73) Titulaire: France Chirurgie Instrumentation, 75015 Paris (FR)
(72) Inventeur: Bruno Fayet, 75007 Paris (FR)
(74) Mandataire: Eidelsberg, Olivier Nathan
(86) Numéro de dépôt international: PCT/FR2007/001770
(87) Numéro de publication internationale: WO 2008/056060

(56) Documents cités:
- EP-A2- 1 048 275
- WO-A-2006/039096
- FR-A- 2 632 531
- US-A- 5 417 651
- US-A1- 2002 151 960

## Description

La présente invention se rapporte à un ensemble d'intubation monocanaliculonasal et/ou monocanaliculaire, destiné notamment à l'imperforation lacrymonasale et aux pathologies canaliculaires.

On connaît déjà, dans l'art antérieur, des ensembles monocanaliculaires qui comportent un mandrin en un matériau sensiblement rigide, par exemple en métal, et un tube en un matériau sensiblement souple, notamment en silicone, fixé à une extrémité du mandrin. A l'autre extrémité du tube en silicone, il est formé un bouchon d'ancrage, dit bouchon méatique, constitué d'une tige s'étendant entre une collerette à une extrémité de la tige et un bulbe à l'autre extrémité de la tige, le bulbe étant relié au tube en silicone et la tige étant sensiblement perpendiculaire à la direction d'extension en longueur du tube.

L'utilisation de ces systèmes connus est la suivante. L'objectif est de déboucher un canal lacrymal bouché. Pour ce faire, on introduit, du côté de l'oeil, l'ensemble monocanaliculaire par le mandrin dans le canal lacrymal jusqu'à venir en contact avec la matière bouchant le canal, celle-ci étant alors percée par le mandrin. Enfin le mandrin en métal finit sensiblement au niveau de l'extrémité nasale du conduit lacrymal, tandis que le tube, qui vient à sa suite, a traversé la matière qui bouchait le canal et permet ainsi un passage à travers celle-ci. L'étape suivante consiste à extraire le mandrin métallique en le tirant hors du nez. Pour ce faire, le chirurgien doit "farfouiller" dans le nez, ce qui peut s'avérer dangereux, notamment dans le cas où l'on opère un nourrisson. Une anesthésie générale, notamment complète, est nécessaire, en particulier avec intubation oro-trachéale ou masque laryngé.

La présente invention vise à surmonter les inconvénients de l'art antérieur en proposant un ensemble d'intubation monocanaliculaire et/ou monocanaliculonasal qui permet au chirurgien d'opérer de manière beaucoup plus sûre et notamment ne l'oblige plus à travailler au niveau du nez du patient pour en extraire le mandrin. Ainsi, en particulier, il n'est plus nécessaire, pour la pose de cette intubation monocanaliculaire destinée à traverser une obstruction dans le canal lacrymal ou canaliculaire en vue de le réouvrir pour le passage des larmes, de pratiquer une anesthésie générale, notamment complète, une simple anesthésie d'inhalation, sans intubation oro-trachéale, étant, suivant l'invention, suffisante.

Au document US 2002/0151960, il est décrit un ensemble suivant le préambule de la revendication 1 ou 2.

Suivant l'invention, l'ensemble d'intubation monocanaliculaire et/ou monocanaliculonasal, est tel que défini à la revendication 1 ou à la revendication 2.

Des perfectionnements avantageux sont décrits aux sous-revendications.

Avec ce nouvel ensemble d'intubation monocanaliculaire, l'utilisation est simplifiée. En effet, maintenant, le chirurgien introduit l'ensemble toujours du côté oeil du canal lacrymal jusqu'à ce que d'une part le bouchon vienne en contact avec un épaulement formé à l'extrémité côté oeil du canal lacrymal, pour ainsi bloquer en mouvement l'ensemble d'intubation, et d'autre part le mandrin, entouré du tube, ait percé la matière obstruant le canal lacrymal ou les voies lacrymales pour permettre la reformation d'une lumière autour du tube en silicone, lumière qui persistera une fois le tube retiré quelque temps après. Pour retirer le mandrin, le chirurgien n'a plus besoin de passer par le nez, ce qui jusqu'à maintenant entraînait des risques importants pour le patient. Le chirurgien appuie sur le canal lacrymal au niveau du mandrin ou maintient le tube par des pinces au niveau du canal et retire simplement par le côté oeil du canal lacrymal le mandrin en le tirant. Il n'est plus maintenant, comme suivant l'art antérieur, sorti par le côté du nez. En outre, il n'est plus non plus nécessaire de tirer l'ensemble jusqu'à la fin du tube en silicone pour amener le bouchon en position. Enfin, une fois que le mandrin a été ressorti, il n'est plus non plus nécessaire de découper le tube, celui-ci ayant dès le départ la dimension souhaitée, ce qui permet d'économiser également en termes de coût de fabrication de l'ensemble d'intubation monocanaliculaire dont la dimension en longueur du tube en silicone est bien plus courte que dans le cas de l'art antérieur.

En particulier, le fait de prévoir un trou dans la partie latérale inférieure de la surface du tube permet d'éviter que l'epithélium canaliculaire subisse une hyperplasie réactionnelle au contact. En effet, dans le cas d'une ouverture réalisée non pas latéralement mais à l'extrémité proximale du tube, l'hyperplasie aurait tendance à épouser tous les contours et interstices et à coloniser le trou débouchant sur une certaine longueur. En outre, une ouverture sous forme de fente à côté du bulbe le respecte complètement. Lorsque l'on retire le mandrin, la fente se collabe et il n'y a pas de colonisation. Enfin, le fait que le bulbe reste fermé limite le risque de canaliculité.

Le bouchon est de préférence constitué d'une tige à une extrémité libre de laquelle se trouve une collerette et à l'autre extrémité côté tube de laquelle se trouve un bulbe, la tige et le tube s'étendant dans deux directions qui se croisent, notamment perpendiculairement.

Suivant un mode de réalisation préféré de l'invention, le bouchon est fixé à une extrémité du tube par insertion d'un bec faisant saillie latéralement de la tige du bouchon et passant à l'intérieur du tube contre la paroi intérieure du tube en silicone, notamment par ajustement serré.

Suivant un mode de réalisation autre préféré de l'invention, le bouchon est issu de la surface latérale du tube et notamment est moulé d'une pièce avec celui-ci.

La présente invention se rapporte également à un ensemble monocanaliculaire et/ou monocanaliculonasal d'intubation comportant un mandrin en un premier matériau sensiblement rigide et un tube en un deuxième matériau moins rigide que le premier matériau, notamment sensiblement souple, un bouchon faisant saillie latéralement du tube, de préférence à une extrémité de celui-ci, destiné à bloquer en position le tube dans un canal lacrymal, caractérisé en ce que le mandrin en le premier matériau a une longueur plus grande que la longueur du tube et une partie du mandrin est insérée à l'intérieur du tube, notamment de manière à ce qu'une de ses extrémités vienne en contact contre l'extrémité fermée du tube à distance du bouchon.

Suivant un mode de réalisation préféré de l'invention, le mandrin a une plus grande épaisseur dans la direction perpendiculaire à la direction de son extension longitudinale, notamment a un diamètre de section transversale, qui est compris(e) entre 0,3 mm et 0,5 mm. Ceci est sensiblement inférieur aux dimensions des mandrins usuellement utilisés dans la technique antérieure.

Suivant un mode de réalisation préféré de l'invention, l'extension en longueur du tube en silicone est comprise entre 15 mm et 60 mm, notamment est égal à 35 mm.

Lorsqu'un chirurgien introduit dans le canal lacrymal, du côté oeil, un ensemble suivant l'invention, le mandrin étant enveloppé en partie par le tube du côté introduit, l'introduction se fait jusqu'à ce que le bouchon vienne en position au niveau du méat lacrymal pour bloquer le tube en position, le mandrin et le tube étant alors disposés dans les voies lacrymales, puis le chirurgien extrait le mandrin en le sortant du même côté oeil par lequel l'introduction s'est faite.

A titre d'exemple, on va maintenant décrire un mode de réalisation préféré de l'invention en se reportant aux dessins dans lesquels :
la figure 1 est une vue en perspective d'un ensemble d'intubation monocanaliculonasal et/ou monocanaliculaire ;
la figure 2, et respectivement 2', est une vue en coupe transversale d'un premier mode de réalisation d'un tube d'un ensemble monocanaliculaire d'intubation suivant l'invention ;
la figure 3 est une vue en coupe longitudinale d'un tube d'intubation monocanaliculaire ;

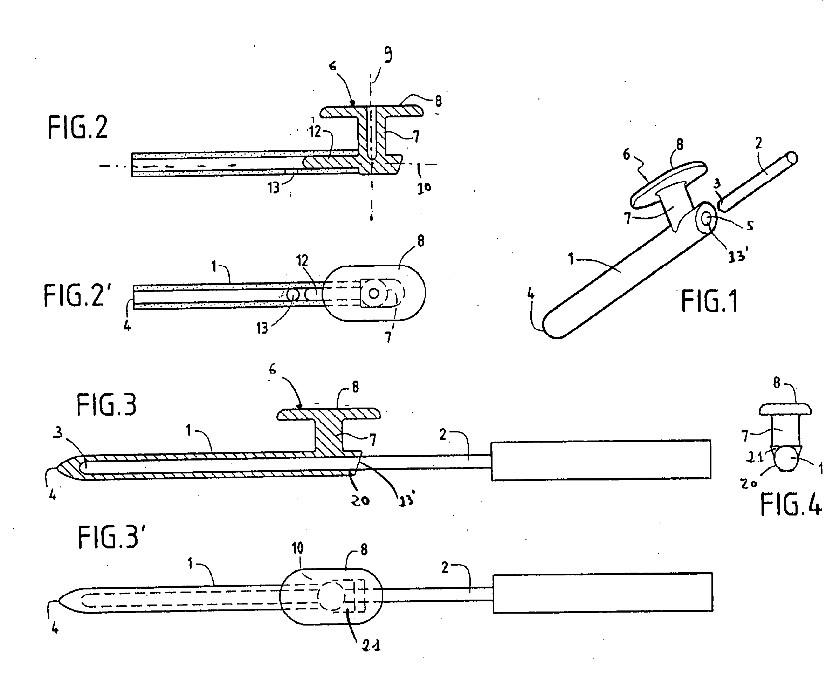

la figure 3' est une vue de dessus du mode de réalisation de la figure 3 ; et
la figure 4 est une vue en coupe au niveau du tronçon 20 du tube des figures 2, 3 et 3'.

A la figure 1, il est représenté, en perspective, un ensemble d'intubation monocanaliculaire et/ou monocanaliculonasal. Celui-ci est constitué, d'une part, d'un tube 1 en silicone borgne et, d'autre part, d'un mandrin 2 en métal. Le mandrin 2 a une extension en longueur supérieure à l'extension en longueur du tube 1, notamment 2 à 4 fois supérieure. Le mandrin 1 est inséré dans le tube 2, de sorte que son extrémité 3 distale borgne soit sensiblement en contact avec la paroi intérieure du tube 1 au niveau de l'extrémité 4 distale de celui-ci. Le tube 1 en silicone comporte, à distance de son extrémité 4 distale, et notamment sensiblement au niveau de son extrémité 5 proximale, un bouchon 6. Ce bouchon 6 est constitué d'une tige ou col 7 et d'une collerette 8 faisant saillie latéralement du col 7 à une extrémité de celle-ci. Le col 7 s'étend suivant un axe longitudinal 9 qui est sensiblement perpendiculaire à l'axe longitudinal du tube 10.

L'extrémité proximale 5 du tube 1 est ouverte (ouverture 13).

Le tube 1 comporte un tronçon 20 qui s'étend entre son extrémité proximale 5 et. la tige 7 et qui a une section transversale plus grande que la section transversale du reste du tube. En particulier, dans ce tronçon 20, le tube comporte des parties d'extension 21 en forme de nervures longitudinales à section triangulaire qui viennent en surplus de la section circulaire du tube.

La fonction du bouchon 6 est la suivante. Une fois que le chirurgien a introduit le tube 1 et le mandrin 2 ensemble (c'est à dire avec une partie du mandrin enveloppée par le tube) à l'extrémité côté oeil des voies lacrymales, le chirurgien poursuit l'avancée de l'ensemble dans les voies lacrymales jusqu'à ce que l'ensemble vienne percer l'obstruction formée dans les voies lacrymales que l'on souhaite percer pour y permettre ultérieurement le passage de larmes, dans un premier temps autour du tube 1 que l'on aura débarrassé du mandrin. L'avancée de l'ensemble est stoppée lorsque le bouchon 6 atteint l'extrémité côté oeil des voies lacrymales et que la collerette 8 vient buter contre l'épaulement ou méat lacrymal formé à l'extrémité du canal lacrymal.

De même, le tronçon 20 de plus grande épaisseur, une fois poussé dans les voies lacrymales, bloque le tube en position pour l'empêcher de ressortir. Cette action du tronçon 20 a pour effet de bloquer le déplacement du tube dans la direction inverse à celle dans laquelle la collerette bloque le déplacement du tube.

La dimension transversale la plus grande, c'est-à-dire ici dans le cas d'un mandrin cylindrique circulaire, le diamètre de la section transversale, du mandrin est de 0,4 mm, et notamment comprise entre 0,3 mm et 0,5 mm.

Suivant un mode de réalisation suivant l'invention représenté à la figure 2, la fixation du bouchon au tube est réalisée par l'insertion d'un bec 12 faisant saillie de la surface latérale de la tige 7 du bouchon et adapté par ajustement serré dans le tube avec collage contre la paroi intérieure du tube 1. On forme alors un trou 13 dans la paroi latérale du tube 1, pour permettre le passage du mandrin.

A la figure 3, le bouchon est réalisé d'une pièce avec le tube, par exemple par moulage, et l'ouverture 13' permettant l'introduction du mandrin 2 est réalisée au niveau de l'extrémité 5 proximale du tube 1.

## Revendications

1. Ensemble d'intubation monocanaliculonasal et/ou monocanaliculaire, destiné notamment à l'imperforation lacrymonasale, comportant un mandrin (2) en un premier matériau sensiblement rigide, par exemple en métal, un tube (1) en un deuxième matériau, moins rigide que le premier matériau, notamment souple, notamment en silicone, un bouchon (6) de blocage, destiné à bloquer en position le tube dans un canal lacrymal, faisant saillie du tube, le bouchon étant constitué d'une tige (7) à une extrémité libre de laquelle se trouve une collerette (8), la tige et le tube s'étendant dans deux directions qui se croisent, notamment perpendiculaires, la longueur du tube (1) étant inférieure à la longueur du mandrin (2), le mandrin (2) ayant une dimension la plus grande en section transversale, notamment un diamètre, mesurée dans la direction perpendiculaire à la direction en longueur, par rapport à celle du tube (1) telle qu'il peut être introduit à l'intérieur du tube et en être retiré, **caractérisé en ce que** le tube (1) comporte, dans sa paroi latérale qui s'étend entre son extrémité distale et la tige du bouchon, un trou (13) pour le passage du mandrin.

2. Ensemble d'intubation monocanaliculonasal et/ou monocanaliculaire, destiné notamment à l'imperforation lacrymonasale, comportant un mandrin (2) en un premier matériau sensiblement rigide , un tube (1) en un deuxième matériau moins rigide que le premier matériau, notamment souple, et un bouchon (6) destiné à bloquer en position le tube dans un canal lacrymal faisant saillie du tube, le bouchon étant constitué d'une tige (7) à une extrémité libre de laquelle se trouve une collerette (8), la tige et le tube s'étendant dans deux directions qui se croisent, notamment perpendiculaires, et le mandrin en le premier matériau ayant une longueur plus grande que la longueur du tube et une partie du mandrin est insérée à l'intérieur du tube par un trou (13) formé dans la paroi latérale du tube entre son extrémité distale et la tige du bouchon.

3. Ensemble suivant la revendication 1 ou 2, **caractérisé en ce que** le tube comporte entre son extrémité proximale (5) et la tige (7) du bouchon un tronçon (20) de plus grande section que le reste du tube.

4. Ensemble suivant l'une des revendications 1 à 3, **caractérisé en ce que** le trou (13) est formé dans la paroi latérale du tube du côté opposé à celui duquel la tige du bouchon s'étend.

5. Ensemble suivant l'une des revendications 1 à 4, **caractérisé en ce que** le trou (13) est formé dans la paroi latérale du tube entre son extrémité (4) distale et la tige (7) du bouchon, à moindre distance de celui-ci que de l'extrémité (4) distale.

6. Ensemble suivant l'une des revendications 2 à 5, **caractérisé en ce que** le mandrin (2) a une plus grande épaisseur en section transversale comprise entre 0,3 mm et 0,5 mm.

7. Ensemble suivant l'une des revendications 1 à 6, **caractérisé en ce que** l'extension en longueur du tube (1) en silicone est comprise entre 15 mm et 60 mm.

8. Ensemble suivant l'une des revendications 2 à 7, **caractérisé en ce que** l'une (3) des extrémités du mandrin vient en contact contre l'extrémité fermée du tube à distance du bouchon.

9. Ensemble suivant l'une des revendications 1 à 8, **caractérisé en ce que** le trou (13) est formé par une fente qui peut se collaber.

## Claims

1. Monocanaliculonasal and/or monocanalicular intubation assembly mainly intended for nasolachrymal imperforation, comprising a mandrel (2) made of a first substantially rigid material, such as a metal, a tube (1) made of a second material that is less rigid than the first material, in particular flexible, such as silicone, a blocking plug (6) for locking the tube in position in the lachrymal duct and protruding from the tube, the plug being formed by a shank (7) at one free end of which is a collar (8), the shank and the tube extending in two directions which cross one another, in particular perpendicular to one another, the length of the tube (1) being smaller than the length of the mandrel (2), the mandrel (2) having a greater dimension in transverse cross section, in particular diameter, measured in the direction perpendicular to the longitudinal direction, relative to that of the tube (1), such that said mandrel can be inserted inside the tube and withdrawn therefrom, **characterized in that** said tube (1) comprises, in its lateral wall, between its distal end and the shank of the plug, a hole (13) for the passage of the mandrel.

2. Monocanaliculonasal and/or monocanalicular intubation assembly notably intended for nasolachrymal imperforation, comprising a mandrel (2) made of a first substantially rigid material, a tube (1) made of a second material that is less rigid than the first material, notably flexible, and a plug (6) for locking the tube in position in a lachrymal duct and protruding from the tube, the plug being formed by a shank (7) at one free end of which is a collar (8), the shank and the tube extending in two directions which cross one another, in particular perpendicular to one another, and the mandrel made of the first - material is longer than the length of the tube and part of the mandrel is inserted inside the tube by a a hole (13) formed in the lateral wall of the tube between its distal end and the shank of the plug.

3. Assembly according to claim 1 or 2, **characterised in that** the tube comprises between its proximal end (5) and the shank (7) of the plug a section (20) with a larger cross section than the rest of the tube.

4. Assembly according to one of claims 1 to 3, **characterised in that** the hole (13) is formed in the lateral wall of the tube on the opposite side to the side from which the shank of the plug extends.

5. Assembly according to one of claims 1 to 4, **characterised in that** the hole (13) is formed in the lateral wall of the tube between its distal end (4) and the shank (7) of the plug, closer to the latter than the distal end (4).

6. Assembly according to one of claims 2 to 5, **characterised in that** the mandrel (2) has a greater thickness in transverse cross section of between 0.3 mm and 0.5 mm.

7. Assembly according to one of claims 1 to 6, **characterised in that** the longitudinal extension of the tube (1) made of silicone is between 15 mm and 60 mm.

8. Assembly according to one of claims 2 to 7, **characterised in that** one (3) of the ends of the mandrel comes into contact with the closed end of the tube remote from the plug.

9. Assembly according to one of claims 1 to 8, **characterised in that** the hole (13) is formed by a slot which is collapsible.

## Patentansprüche

1. Monocanaliculonasale und/oder monocanaliculare Intubationsanordnung, die insbesondere für die nasolakrimale Imperforation bestimmt ist und einen Mandrin (2) aus einem ersten im Wesentlichen steifen Material, zum Beispiel aus Metall, aufweist, eine Röhre (1) aus einem zweiten Material, weniger steif als das erste Material, insbesondere flexibel, insbesondere aus Silikon, einen Blockierstopfen (6), der dafür bestimmt ist, die Röhre in einem Tränenkanälchen in der Position zu blockieren und aus der Röhre ragt, wobei der Stopfen aus einem Schaft (7) gebildet ist, wobei sich an einem freien Ende davon ein Bund (8) befindet, wobei sich der Schaft und die Röhre in zwei Richtungen erstrecken, die sich, insbesondere senkrecht, kreuzen, wobei die Länge der Röhre (1) geringer ist als die Länge des Mandrins (2), wobei der Mandrin (2) eine größte Abmessung im Querschnitt, insbesondere einen Durchmesser, in der Richtung senkrecht zur Längsrichtung gemessen aufweist, bezogen auf die der Röhre (1), sodass er ins Innere der Röhre eingeführt und aus ihr herausgezogen werden kann, **dadurch gekennzeichnet, dass** die Röhre (1), in ihrer Seitenwand, die zwischen ihrem distalen Ende und dem Schaft des Stopfens verläuft, eine Öffnung (13) zum Durchführen des Mandrins aufweist.

2. Monocanaliculonasale und/oder monocanaliculare Intubationsanordnung, die insbesondere für die nasolakrimale Imperforation bestimmt ist, die einen Mandrin (2) aus einem ersten im Wesentlichen steifen Material aufweist, eine Röhre (1) aus einem zweiten Material, weniger steif als das erste Material, insbesondere flexibel, und einen Stopfen (6), der dafür bestimmt ist, die Röhre in einem Tränenkanälchen in der Position zu blockieren und aus der Röhre ragt, wobei der Stopfen aus einem Schaft (7) gebildet ist, wobei sich an einem freien Ende davon ein Bund (8) befindet, wobei sich der Schaft und die Röhre in zwei Richtungen erstrecken, die sich, insbesondere senkrecht, kreuzen, und der Mandrin aus dem ersten Material eine Länge aufweist, die größer ist als die Länge der Röhre, und ein Teil des Mandrins über eine Öffnung (13), die in der Seitenwand der Röhre zwischen ihrem distalen Ende und dem Schaft des Stopfens ausgebildet ist, ins Innere der Röhre eingeführt ist.

3. Anordnung nach Anspruch 1 oder 2, **dadurch gekennzeichnet, dass** die Röhre zwischen ihrem proximalen Ende (5) und dem Schaft (7) des Stopfens einen Teilabschnitt (20) mit einem größeren Querschnitt als die übrige Röhre aufweist.

4. Anordnung nach einem der Ansprüche 1 bis 3, **dadurch gekennzeichnet, dass** die Öffnung (13) in der Seitenwand der Röhre auf der Seite gegenüber der, von der aus der Schaft des Stopfens verläuft, gebildet ist.

5. Anordnung nach einem der Ansprüche 1 bis 4, **dadurch gekennzeichnet, dass** die Öffnung (13) in der Seitenwand der Röhre zwischen ihrem distalen Ende (4) und dem Schaft (7) des Stopfens gebildet ist, in einem geringeren Abstand zu diesem als zum distalen Ende (4).

6. Anordnung nach einem der Ansprüche 2 bis 5, **dadurch gekennzeichnet, dass** der Mandrin (2) eine größere Dicke im Querschnitt zwischen 0,3 mm und 0,5 mm aufweist.

7. Anordnung nach einem der Ansprüche 1 bis 6, **dadurch gekennzeichnet, dass** die Längserstreckung der Röhre (1) aus Silikon zwischen 15 mm und 60 mm beträgt.

8. Anordnung nach einem der Ansprüche 2 bis 7, **dadurch gekennzeichnet, dass** eins (3) der Enden des Mandrins das geschlossene Ende der Röhre entfernt vom Stopfen berührt.

9. Anordnung nach einem der Ansprüche 1 bis 8, **dadurch gekennzeichnet, dass** die Öffnung (13) durch einen Schlitz gebildet ist, der sich zusammenlegen kann.
